# EUROPÄISCHE PATENTSCHRIFT

(11) **EP 1 702 078 B1**
(45) Veröffentlichungstag und Bekanntmachung des Hinweises auf die Patenterteilung: **03.03.2010**
(21) Anmeldenummer: 04802928.4
(22) Anmeldetag: 13.12.2004
(51) Int. Cl.: C12Q 1/68

(54) **TIERARTSPEZIFISCHER UND QUANTITATIVER NACHWEIS VON ZNS-GEWEBE IN FLEISCH UND FLEISCHERZEUGNISSEN**
ANIMAL SPECIES-SPECIFIC AND QUANTITATIVE DETECTION OF CENTRAL NERVOUS SYSTEM TISSUE IN MEAT AND MEAT PRODUCTS
DETECTION QUANTITATIVE ET SPECIFIQUE D'UNE ESPECE ANIMALE DE TISSUS DU SYSTEME NERVEUX CENTRAL DANS DE LA VIANDE ET DES PRODUITS CARNES

(30) Priorität: 23.12.2003 DE 10361489
(43) Veröffentlichungstag der Anmeldung: 20.09.2006
(73) Patentinhaber: Justus-Liebig-Universität Giessen, 35390 Giessen (DE)
(72) Erfinder: BÜLTE, Michael, 35305 Reinhardshain (DE); SCHÖNENBRÜCHER, Holger, 35398 Giessen (DE); ABDULMAWJOOD, Amir, 35396 Giessen (DE)
(74) Vertreter: Buchhold, Jürgen
(86) Internationale Anmeldenummer: PCT/DE2004/002723
(87) Internationale Veröffentlichungsnummer: WO 2005/061726

(56) Entgegenhaltungen:
- WO-A-99/50661
- SEYBOLDT C ET AL: "Reverse transcription-polymerase chain reaction assay for species-specific detection of bovine central nervous system tissue in meat and meat products." JOURNAL OF FOOD PROTECTION, Bd. 66, Nr. 4, April 2003 (2003-04), Seiten 644-651, XP008048368 ISSN: 0362-028X
- LANGE BIANCA ET AL: "[Molecular biological detection of tissues of central nervous system in meat products]" BERLINER UND MUNCHENER TIERARZTLICHE WOCHENSCHRIFT. 2003 NOV-DEC, Bd. 116, Nr. 11-12, November 2003 (2003-11), Seiten 467-473, XP008048367 ISSN: 0005-9366
- SCHMIDT G R ET AL: "The detection of central nervous system tissue on beef carcasses and in comminuted beef" JOURNAL OF FOOD PROTECTION, Bd. 64, Nr. 12, Dezember 2001 (2001-12), Seiten 2047-2052, XP008048393 ISSN: 0362-028X
- AGAZZI MARIE-ELISABETH ET AL: "Performance comparison of two analytical methods for the detection of tissues of the central nervous system in sausages: Results of an interlaboratory study" EUROPEAN FOOD RESEARCH AND TECHNOLOGY, Bd. 215, Nr. 4, Oktober 2002 (2002-10), Seiten 334-339, XP008048350 ISSN: 1438-2377
- TANGA F Y ET AL: "Real time RT - PCR spinal assessment of the temporal regulation of glial activation and proinflammatory cytokines in a rat model of neuropathy." SOCIETY FOR NEUROSCIENCE ABSTRACT VIEWER AND ITINERARY PLANNER, Bd. 2003, 2003, Seiten Abstract No. 696.19 URL-http://sf, XP008048399 & 33RD ANNUAL MEETING OF THE SOCIETY OF NEUROSCIENCE; NEW ORLEANS, LA, USA; NOVEMBER 08-12, 2003
- JELASO A M ET AL: "Exposure to PCBs causes suppression of neural - immune response genes in C6 glioblastoma cells." SOCIETY FOR NEUROSCIENCE ABSTRACT VIEWER AND ITINERARY PLANNER, Bd. 2003, 2003, Seiten Abstract No. 710.12 URL-http://sf, XP008048400 & 33RD ANNUAL MEETING OF THE SOCIETY OF NEUROSCIENCE; NEW ORLEANS, LA, USA; NOVEMBER 08-12, 2003
- BECKER ALBERT J ET AL: "Transcriptional profiling in human epilepsy: expression array and single cell real-time qRT-PCR analysis reveal distinct cellular gene regulation." NEUROREPORT. 19 JUL 2002, Bd. 13, Nr. 10, 19. Juli 2002 (2002-07-19), Seiten 1327-1333, XP008048414 ISSN: 0959-4965

## Beschreibung

### Stand der Tecknik

Seit dem Bekanntwerden erster Fälle der Bovinen Spongiformen Encephalopathie (BSE) 1985 im Vereinigten Königreich bei Milchkühen sind circa 5 Mio. Rinder aufgrund einer bereits bestehenden Infektion oder im Rahmen von präventiven Maßnahmen getötet worden. Der wirtschaftliche Schaden beläuft sich bislang allein innerhalb des Vereinigten Königreiches auf über $ 6 Millionen (SMITH, 2003).

Die BSE-Erkrankung zählt zum Formenkreis der Transmissiblen Spongiformen Encephalopathien (TSE), die durchs fehlgefaltete Eiweißkörper, sog. Prionen (PrP^{sc}), beim Menschen sowie bei einer Vielzahl von Säugetieren auftreten können. Es gibt mittlerweile keinen wissenschaftlichen Zweifel mehr daran, daß es sich bei der beim Menschen auftretenden neuen Variante der Creutzfeld Jakob Disease (vCJD), um die humane Form der BSE handelt (COLLINGE et al.; 1996; DEALLER, 1998). Trotz intensiver Forschung und erheblichen Anstrengungen sind nach derzeitigem wissenschaftlichen Kenntnisstand alle Formen der TSE-Erkrankungen nicht therapierbar und stets tödlich verlaufend. Neben diesem, auf der "International Prion Conference" im Oktober 2003 in München gezogenen Fazit kommt erschwerend hinzu, daß bislang keine diagnostischen Systeme zur Verfügung stehen, die die Detektion einer Infektion des Menschen oder der Tiere vor dem Auftreten klinisch apparenter Erkrankungen zuverlässig ermöglichen. Man ist nach wie vor auf die postmortale Diagnose zur Verifizierung einer Erkrankung dieses Formenkreises angewiesen.

Das kausative Agens, die pathomorphologische Form des Prionenproteins (PrP^{sc}) gilt als Auslöser einer Vielzahl weiterer beim Tier vorkommender TSE-Erkrankungen. Dabei handelt es sich z.B. um die hauptsächlich bei Schafen auftretende Scrapie und die bei nordamerikanischen Hirscharten sowie Elchen endemisch vorkommende Chronic Wasting Disease (CWD). Der epidemiologische Zusammenhang der einzelnen TSE-Formen untereinander und die nicht mehr auszuschließende Übertragbarkeit auf den Menschen ist Gegenstand einer Vielzahl gegenwärtiger Untersuchungen. In diesem Zusammenhang soll darauf hingewiesen werden, daß in neuen Versuchsreihen die Übertragbarkeit einer für Nerze pathogenen PrP^{sc}-Variante auf das sog. humane Mausmodell mit dem Ergebnis gezeigt werden konnte, daß bei den Tieren auch die konventionelle Form der CJD ausgelöst wurde (SIGURDSON und MILLER, 1998; COLLINGE, 2003). Die pathogenetische Entwicklung der BSE schließt einen oralen Infektionsweg über die Zunge ebenso ein, wie die PrP^{sc}-Replikation in bestimmten Organen, v. a. der Milz (LASMÉZAS et al., 2003). Vor dem Hintergrund eines präventiven Lebensmittelsicherheitsmanagements ergibt sich trotz sinkender BSE-Fallzahlen in der Europäischen Gemeinschaft nach wie vor ein erheblicher und breit angelegter Forschungsbedarf.

Die bislang effektivste Möglichkeit zum Schutz des Menschen vor der Übertragung einer prionenbasierten, vom Tier ausgehenden Erkrankung (Zoonose) ist daher der Ausschluss der als hochinfektiös einzustufenden entsprechenden tierischen Gewebe, dem sogenannten Spezifizierten Risikomaterial (SRM) aus der Lebensmittelproduktion. In der Verordnung der Europäischen Gemeinschaft (EG) Nr. 999/2001 in der Fassung vom 22. Mai 2001 mit Vorschriften zur Verhütung, Kontrolle und Tilgung bestimmter transmissibler spongiformer Encephalopathien; zuletzt geändert durch die Verordnung (EG) Nr. 1139/2003 der Kommission vom 27. Juni 2003 (ABI. L 160, Seite 22-32) mit Gültigkeit ab dem 1. Oktober 2003, sind folgende Gewebe als spezifiziertes Risikomaterial definiert:

Bei über 12 Monate alten Rindern: Schädel ohne Unterkiefer, aber einschließlich Gehirn und Augen, Wirbelsäule ohne Schwanzwirbel, Querfortsätze der Lenden- und Brustwirbel sowie Kreuzbeinflügel, aber einschließlich der Spinalganglien und des Rückenmarks sowie Tonsillen, Darm von Duodenum bis Rektum und Mesenterium von Rindern aller Altersklassen.

Bei Schafen und Ziegen, die über 12 Monate alt sind bzw. bei denen ein bleibender Schneidezahn das Zahnfleisch durchbrochen hat: Schädel, einschließlich Gehirn und Augen, Tonsillen und Rückenmark sowie bei Schafen und Ziegen aller Altersklassen Milz und Ileum. Aus der Auflistung wird ersichtlich, daß es sich bei dem SRM in erster Linie um zentralnervöses Gewebe handelt.

Es ist weiterhin zu bedenken, daß die gewerbliche Vermarktung von Kopffleisch der Tierart Rind als Lebensmittel, aus primär ökonomischen Erwägungen, seit dem 1. Oktober 2003 wieder freigegeben worden ist. Aufgrund der schlachttechnologischen Herrichtung der Rindertierkörper besteht die Möglichkeit einer Kontamination mit ZNS-Gewebe und damit die Gefahr einer Einschleppung in die menschliche Nahrungsmittelkette. Von den zuständigen Behörden wird daher grundsätzlich bei der Zulassung von Betrieben zur Gewinnung von Kopffleisch die regelmäßige Kontrolle auf ZNS-Kontamination zur Auflage gemacht und eingefordert.

Die Entwicklung und zur Verfügungstellung von Verfahren zum schnellen und zweifelsfreien Nachweis von Risiko-Gewebe in der menschlichen, aber selbstverständlich auch tierischen Nahrungsmittelkette ist daher zwingend geboten. Aus den fleischhygienerechtlichen Erfordernissen zur Entfernung und Beseitigung von TSE-Risikomaterial ergibt sich die tierartspezifische Ausrichtung solcher Nachweissysteme. Gleichzeitig erscheint eine quantitative Erfassung von SRM wünschenswert, um die Art einer Kontamination, so z.B. die geringgradige, auch als Verschleppungskontamination zu charakterisierende Verunreinigung, im Gegensatz zu der vorsätzlichen Einarbeitung oder fahrlässigen Kontamination von ZNS-Gewebe in das Lebensmittel abgrenzen zu können. Das Gehirn besitzt hervorragende emulgatorische Eigenschaften, und ist daher - vor dem Verbot - häufiger bei der Herstellung von bestimmten Fleischprodukten (v. a. brühwurstartiger Erzeugnisse), im Vereinigten Königreich insbesondere zur Produktion von "Beefburgern" technologisch (bis zu 10% anteilig) genutzt worden.

In diesem Zusammenhang ist von Bedeutung, daß die Verarbeitung von Schweinehirn fleischhygienerechtlich nicht untersagt ist, ebenso wenig wie die Verarbeitung von Geflügelseparatorenfleisch. Dieses stammt überwiegend von mechanisch separierten Putenwirbelsäulen mit entsprechender Kontamination durch das Rückenmark. Allerdings wird in den Leitsätzen für Fleisch und Fleischerzeugnisse - ohne explizite Angabe der Tierart - darauf hingewiesen, daß Him und Rückenmark nicht in Fleischerzeugnissen verarbeitet werden (LS für Fleisch und Fleischerzeugnisse, zuletzt geändert am 2.10.2001) (BAnz. Nr. 199 vom 24. 10. 2001, GMBI Nr. 38 S. 754 ff vom 30.10. 2001). Dieses wird in der Bundesrepublik in der industriellen Produktion sicherlich eingehalten; in registrierten, d. h. kleineren Betrieben (z. B. Metzgereien, Direktvermarkter) ist dieses möglicherweise nicht bekannt oder wird weniger stringent ausgelegt und gehandhabt. In osteuropäischen Ländern hingegen ist die Verarbeitung von Him jedoch regional noch weit verbreitet (EU-Twinning Projekt, Slowakei, 2000).

Bislang stehen ausschließlich tierartunspezifische und phänotypische Verfahren zum Nachweis von ZNS zur Verfügung (LÜCKER et. al., 1997, 1998, 1999 und SCHMIDT et al., 2001) Bei diesen Untersuchungen wurde vor allem die Eignung des sauren Gliafaserproteins (GFAP) als Marker für den Nachweis von ZNS-Gewebe aus Lebensmitteln geprüft. Auf diesem Marker basierende Westemblot- bzw. ELISA-Techniken (SCHMIDT et al., 2001) ermöglichen allerdings weder eine Spezies-spezifische Zuordnung noch die sichere Bereitstellung quantitativer Daten.

Die diesbezüglichen Patentschriften DE 198 14 088 C1 und DE 198 04 216 C2 beinhalten Verfahren zum Nachweis von zentralnervösern Gewebe in Erzeugnissen, insbesondere in Fleischerzeugnissen, sowie Testkits hierzu. Beide Patentschriften nehmen Bezug auf die Möglichkeit einer quantitativen Nachweisführung. Dabei handelt es sich allerdings ausschließlich um phänotypische Verfahren. Die wesentlichen Nachteile der darin beschriebenen tierartunspezifischen Quantifizierung von ZNS-Gewebe sind:

Es erfolgt eine rein visuelle Auswertung seitens der Versuchsdurchführenden, d.h., dass die Signalstärke der untersuchten Probe mit der hausinterner Standards abgeglichen werden muss. Seitens des Laborpersonals wird somit ein hohes Maß an Einarbeitung in die entsprechende Methode vorausgesetzt. Gleichzeitig weisen die verwendeten Marker aufgrund ihrer differerierenden Gewebespezifität eine sehr unterschiedliche Eignung für die Quantifizierung von ZNS-Gewebe in Fleisch und Fleischerzeugnissen auf. Allen mit der Thematik Vertrauten ist bekannt, dass ein GFAP-Westemblot für eine vergleichende visuelle Quantifizierung allenfalls nur bedingt geeignet ist. Das ist vor allem durch das Auftreten verschiedener GFAP-Bandenmuster, mit dann auch noch variierender Intensität der einzelnen Banden, bedingt, die sowohl innerhalb ein und desselben Versuchsansatzes als auch im Vergleich zum erforderlichen Referenzmaterial in verschiedenen Versuchsansätzen deutlich voneinander abweichen können.

Die von LÜCKER und BÜLTE, (1997) vorgestellte enzymatische Bestimmung des Cholesterolgehaltes muß als allenfalls bedingt geeignet angesehen werden, da hohe Cholesterolgehalte, z.B. in der Leber, zu falsch-positiven Signalen führen können.

Unabhängig von einer beabsichtigten Quantifizierung ist zu berücksichtigen, daß die Kombination mehrerer Markerproteine erforderlich ist. So ist z.B. die Verwendung der Neuronenspezifischen Enolase (NSE) lediglich als unspezifische Screeningreaktion zu werten. Die ZNS-Spezifität des Ergebnisses muss in einer zweiten Reaktion mittels GFAP bestätigt werden, wobei aber kein tierartspezifischer Nachweis möglich ist.

Auch die vorhandene ELISA-Methode (SCHMIDT et al., 2001) bzw. in der Formulierung als RIDASCREEN^{®} Risk Material, Enzymimmunoassay zur semiquantitativen Bestimmung von Risikomaterial (r-biopharm), Art. No.: R6701 erlaubt bislang keine sichere Quantifizierung. Die Festlegung benötigter Cutoff-Wert muss als unausgereift bezeichnet werden. Entsprechende Ergebnisse eigener Untersuchungen belegen allenfalls eine grob orientierende Abschätzung .

Zum Nachweis von mRNA sind neben der Real Time Reverse Transkription-PCR (Tanga, F.Y. et al., 2003 und Becker et al., 2002) RT-PCR-Verfahren zur Detektion von GFAP mRNA bekannt.

So beschreiben Seyboldt et al. (2003) ein Verfahren zur Detektion von GFAP mRNA aus bovinem ZNS-Gewebe in Fleischprodukten. Dabei wird zunächst eine reverse Transkription der kompletten extrahierten RNA mit Random Hexameren durchgeführt und anschließend die erhaltene cDNA mit den Primern GFPAfor und GFAPrev amplifiziert. Die so erhaltenen Amplifikationsprodukte der GFAP mRNA werden dann einer RFLP-Analyse unterzogen. Aufgrund der unterschiedlichen Fragmentlänge können dann die Amplifikate einer bestimmten Spezies zugeordnet werden.

Auch Lange et al. (2003) beziehen sich auf den Nachweis von ZNS in Fleischerzeugnissen nach reverser Transkription mittels PCR (RT-PCR). Als Markerproteine werden dabei GFAP und das basische Myelinprotein (MBP) verwendet.

Bei allen diesen Verfahren können jedoch keine zuverlässigen Aussagen über die Tierart- bzw. Organspezifität des nachzuweisenden ZNS-Materials getroffen werden. Darüber hinaus können in beträchtlichem Umfang falsch-positive Signale auftreten.

### Literatur

BECKER, A. J., Chen, J., Paus, S., Normann, S., Beck, H., Elger, C. E., Wiestler, O. D., Blümcke, I. (2002): Transcripional profiling in human epilepsy: expression array and single cell real-time qRT-PCR analysis reveal distinct cellular gene regulation. Neuroreport, Vol. 13 No. 10.
COLLINGE, J., SIDLE, K., MEADS, J., IRONSIDE, J. and HILL, A. (1996): Molecular analysis of prion strain variation and the aetiology of 'new variant 'CJD. Nature 383: 685-690
COLLINGE, J. (2003): Prion propagation, strains and interspecies transmission. Abstr. Conf.: Prion diseases: from basic research to intervention concepts. München, 08.10. bis 10.10.2003
Condorelli, D. F., Nicoletti, V. G., Barresi, V., Ponticello, S. G., Caruso, A., Tendi, E. A., Giuffrida Stella, A.M. (1999):Structural features of the rat GFAP gene and identification of a novel alternative transcript. J. Neurosci. Res. 56 (3):219-228.
DEALLER, S. (1998): Post-exposure prophylaxis after accidental prion inoculation. Lancet Feb. 21: 351 (9102), Comment on: Lancet 1997 Nov. 22; 350 (9090): 1519-1520
EU-Twinning Projekt SR 88/AG 01 Hessen/Slowakei im Jahr 2000
GUERTLER, M., ALTER, T., FROEB, A., LANGE, B., JOHNE, R., LUECKER, E. and FEHLHABER, K. (2003): Nucleotide sequence of the bovine glial fibrillary acidic protein (GFAP) gene. Direct submission to Genbank, 14.01.2003
LANGE, B., Alter, T., Froeb, A., Lücker, E. (2003): Molekularbiologische Erfassung von Geweben des Zentralen Nervensystems in Fleischerzeugnissen. Berl. Münch. Tierärztl. Wschr. 116, 467-473.
LASMEZAS, C. I., HERZOG, C., ETCHEGARAY, N., DORMONT, D. and DESLYS, J. P. (2003): Pathogenesis of BSE in non-human primates. Abstr. Conf.: Prion diseases: from basic research to intervention concepts. München, 08.10. bis 10.10.2003
Leitsätze für Fleisch und Fleischerzeugnisse, zuletzt geändert am 2.10.2001 (BAnz. Nr. 199 vom 24. 10. 2001, GMBI Nr. 38 S. 754 ff vom 30.10. 2001)
LÜCKER, E. und BÜLTE, M. (1997): Verfahren zum Nachweis von im Hinblick auf die bovine spongiforme Enzephalopathie (BSE) unerwünschten Zutaten in Fleischerzeugnissen. 1. Enzymatische Cholesterinbestimmung als Schnellverfahren zur Erfassung von Hirngewebe. Fleischwirtschaft 77: 836-840
LÜCKER E., EIGENBRODT, E. und BÜLTE, M. (1998): Verfahren zum Nachweis von im Hinblick auf die bovine spongiforme Enzephalopathie (BSE) unerwünschten Zutaten in Fleischerzeugnissen. 2. Referenzverfahren für den Nachweis von zentralem Nervengewebe. Fleischwirtschaft 78: 896-898
LÜCKER, E., EIGENBRODT, E., WENISCH, S., FALING, M., LEISER R. and BÜLTE, M. (1999): Development of an integrated procedure for the detection of central nervous tissue in meat products using cholesterol and NSE as markers. J. Food Prot. 62 (3): 268-276
Nielsen, A.L., Holm, I.E., Johansen, M., Bonven, B., Jorgensen, P., Jorgensen, A.L. (2002): A new splice variant of glial fibrillary acidic protein, GFAP epsilon, interacts with the presenilin proteins. J. Biol. Chem. 277 (33):29983-29991.
SCHMIDT, G. R., YEMM, R. S., CHILDS, K. D., O'CALLGHAN, J. P. and HOSS-NER, K. L. (2001): The Detection of Central Nervous System Tissue on Beef Carcasses and in Comminuted Beef. J. Food Prot. 64 (12): 2047-2052
SEYBOLDT, C., John, A., v. Mueffling, T., Nowak, B. and Wenzel,S. (2003): Reverse Transcription-Polymerase Chain Reaction Assay for Species-Specific Detection of Bovine Central Nervous System Tissue in Meat and Meat Products. Journal of Food Protection, Vol. 66, No.4, 2003, pages 644-651.
SIGURDSON, C. J. and MILLER, M. W. (2003): Other animal prion diseases. Br. Med. Bull. 66:199-212
SMITH, P. G. and BRADLEY, R. (2003): Bovine spongiform encephalopathy (BSE) and its epidemiology. Br. Med. Bull. 66:185-98
TANGA, F.Y., DeLeo, J.A. (2003): Real Time RT-PCR Spinal Assesment of the Temporal Regulation of Glial Activation and Proinflammatory Cytokines in a Rat Model of Neuropathy. Program No. 696.19 2003 Abstract Viewer/ltinerary Planner. Washington, DC: Society for Neuroscience, 2003.
Verordnung der Europäischen Gemeinschaft (EG) Nr. 999/2001 in der Fassung vom 22. Mai 2001 mit Vorschriften zur Verhütung, Kontrolle und Tilgung bestimmter transmissibler spongiformer Encephalopathien, zuletzt geändert durch die Verordnung (EG) Nr. 1139/2003 der Kommission vom 27. Juni 2003 (ABI. L 160, Seite 22-32) mit Gültigkeit ab dem 1. Oktober 2003
YU, M., ZHAO, S., LIU, B., XIONG, T., PAN, P. and LI, K. (2001): Rapid communication: isolation and regional localization of the porcine glial fibrillary acidic protein (GFAP) gene. J. Anim. Sci. 79 (10). 2754

### Aufgabe

Aufgabe der vorliegenden Erfindung ist es, die beschriebenen Nachteile im Stand der Technik zu beheben und ein Verfahren zum Nachweis von ZNS-Gewebe in Fleisch und Fleischerzeugnissen bereitzustellen, das eine tierartspezifische und quantitative Auswertung ermöglicht, einfach, sicher und auch erfolgreich mit hitze-behandeltem Fleisch und Fleischerzeugnissen durchführbar ist.

### Lösung der Aufgabe

Diese Aufgabe wird erfindungsgemäß gelöst durch ein tierartspezifisches und quantitatives Verfahren zum Nachweis von ZNS-Gewebe in Fleisch und Fleischerzeugnissen mittels saurer Gliafaserprotein (GFAP)- messenger (m)-RNA, und einem entsprechenden Testkit zur Durchführung des Verfahrens gemäß der Ansprüche.

Das erfindungsgemäße Verfahren weist selektiv die Existenz von ZNS-Gewebe in Fleisch und Fleischerzeugnissen aus den Tierarten Rind, Schaf und Ziege und alternativ auch aus der Tierart Schwein nach und es ermöglicht im gleichen Verfahrensgang die quantitative Auswertung durch die Real Time-PCR unter Erfassung von saurer Gliafaserprotein (GFAP)- messenger (m)-RNA. Der spezifische Nachweis erfolgt auch in hitzebehandeltern Fleisch und Fleischerzeugnissen.

Das erfindungsgemäße Verfahren besteht im Einzelnen aus folgenden Schritten
a) Aufbereitung des Probenmaterials und RNA-Extraktion
b) Reverse Transkription der RNA in cDNA
c) Analyse der cDNA in der Real Time-PCR, wobei die folgenden Primer verwendet werden:

| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3' |

Das erfindungsgemäße Verfahren stellt ein genotypisches Nachweisverfahren für ZNS-Material dar. Es erlaubt die Detektion von kleinsten RNA-Spuren und weist damit selbst geringfügige ZNS-Kontaminationen in Risikomaterial und anderen biologischen Proben nach.

Erstmals ist eine tierartspezifische Zuordnung möglich, d.h. es wird dargestellt, ob das ZNS-Material von den Tierarten Rind, Schaf und Ziege oder vom Schwein stammt. Darüber hinaus erfolgt im gleichen Verfahrensschritt eine exakte Quantifizierung der detektierten GFAP-cDNA- respektive GFAP-RNA-Gehalte der genannten Tierarten.

Insgesamt ermöglicht das Verfahren den quantitativen Nachweis und die tierartspezifische Differenzierung von Verschleppungs- bzw. bewußt oder fahrlässig herbeigeführten Einarbeitungen von sog. Risikomaterialien in das Lebensmittel auf schnelle und sichere Art und Weise.

Im Vergleich zu den bekannten phänotypischen Verfahren, die ausschließlich tierartunspezifisch und auf Proteinbasis fungieren, erlaubt die Kombination aus Reverser Transkriptase (RT)-PCR und der Real Time-Technologie unter Einsatz des TaqMan-Prinzips einen deutlich sensitiveren Verfahrensgang, mit dem bis zu 0,01 % ZNS-Gewebe auch in hitzebehandelten, analytisch als besonders problematisch einzustufende Erzeugnissen, nachgewiesen werden. Die Nachweisgrenze des Real Time-PCR-Systems, die nach einer dem Fachmann geläufigen Methoden über die Analyse dekadischer Verdünnungsreihen ermittelt wurde, liegt bei circa 1,0 x 10⁻¹² g/PCR.

Der wesentliche Vorteil der Real Time-PCR liegt im Vergleich zur konventionellen PCR in der um den Faktor 30 höheren Sensitivität und der Möglichkeit zur direkten Quantifizierung. Dem Fachmann ist zudem ersichtlich, daß durch die zeitauflösenden und parallelen Messungen die arbeits- und zeitintensive gelelektrophoretische Auswertung nicht mehr notwendig ist.
Unabhängig von der Probenmatrix und dem vorliegenden Prozessierungs- bzw. Erhitzungsgrad wird die zuverlässige Nachweisführung in Verbindung mit der Probenaufbereitung in weniger als 8 Stunden ("one day result") erreicht.

Zusammen mit den optimierten und für diese Anwendung in der beschriebenen Kombination neuartigen Probenaufbereitungsverfahren wird ein zuverlässiges und sicher arbeitendes Verfahren zur Verfügung gestellt, dessen Durchführung und einfache Auswertung auch von weniger gut ausgebildetem Laborpersonal möglich ist.

Das Grundprinzip des erfindungsgemäßen Verfahren ist die selektive Erfassung von cDNA. Methodische Basisvoraussetzung für eine sichere Verfahrensdurchführung ist daher die Bereitstellung einer qualitativ und quantitativ hochwertigen cDNA-Probe, dies wird erfindungsgemäß durch ein Two-Step-Prinzip realisiert. Darüber hinaus werden die Vorteile des Reverse-Transkriptase-Systems als das zur Zeit empfindlichste wissenschaftliche Verfahren zum RNA-Nachweis genutzt und durch die Verwendung von Random Hexameren eine sichere Transkription der gesamten isolierten RNA inklusive Sekundärstrukturreicher Regionen ermöglicht. Besonders vorteilhaft ist die geringe Größe des mittels der Real-Time-PCR analysierten Genabschnittes bzw. der cDNA-Region. Die mRNA-Zielregion ist über mindestens 2 Jahre auch in erhitzten Fleisch und Fleischerzeugnissen stabil und damit sicher detektier- und quantifizierbar.

Zusätzliche qualitätssichemde Maßnahmen, z.B. das Arbeiten im geschlossenen System (d.h. Homogenisation und Lyse der RNA erfolgen in einem Gefäß) und DNAse-Verdau sichern gegen evtl. DNA-Köntaminationen von Seiten der Probenaufbereitung und der Umschreibung der RNA ab und verhindern mögliche Fehlerquellen, die durch das Laborpersonal bedingt sein können.

Damit ist das neue Verfahren allen bisherigen Methoden eindeutig überlegen.

### Ausführungsbeispiele

### a) Aufbereitung des Probenmaterials und RNA-Extraktion

Als Probenmaterialien, d.h. zu untersuchende Probe oder Kontrollprobe werden beispielsweise Gehirn, Rückenmark, Fleisch und Fleischerzeugnisse teilweise mit hohem Fettsäuregehalt eingesetzt. Aufgrund der Vielschichtigkeit der zu untersuchenden Fleisch und Fleischerzeugnisse (z.B. Rohwürste, Brühwurstkonserven oder native Kopffleischproben) ist der Homogenisierungsschritt wichtig, der so optimiert ist, daß Kontaminationen vermieden werden.

Neben zahlreichen, dem Fachmann bekannten Standardverfahren zur Probenaufbereitung in der Lebensmittelanalytik, wie der Verwendung der Schwingmühle, des Elvehjem-Potters, des Ultra Turrax T25 (Fa. Janke & Kunkel, Staufen), wird vorzugsweise die besonders effiziente und zeitsparende Homogenisation mittels Fast-Prep-120 durchgeführt. Hier besteht der Homogenisationsschritt aus einer Kombination vertikaler Rotations- und horizontaler Auf- und Ab-Bewegungen. Dadurch sind Geräte dieser Art besonders gut geeignet, um den seitens der heterogenen Probematrizes bestehenden Anforderungen gerecht zu werden.

Da das Probenmaterial insbesondere bei enthaltenem ZNS-Gewebe einen besonders hohen Fettsäuregehalt aufweist, werden an die RNA-Extraktion besondere Anforderungen gestellt.

Die RNA-Extraktion erfolgt prinzipiell mit einer der bekannten Standardmethoden. Diese sind dem Fachmann bekannt und in Laborstandardwerken, wie Sambrook, Fritsch, Maniatis, 1989, Molecular Cloning, CSH Laboratory Press, Cold Spring Harbour, NY. etc ausführlich beschrieben. Hauptmerkmal ist die Guanidinthiocyanat-Phenol gestützte RNA-Extraktion.

Vorzugsweise wird zur Gewinnung der gesamten zellulären RNA der RNeasy^{®} Lipid Tissue mini Kit (Qiagen, Darmstadt) eingesetzt. Der wesentliche Vorteil dieses Verfahrens besteht in der Vorschaltung einer Lyse und Extraktion auf Phenolbasis, der die zuverlässige RNA-Extraktion aus Matrizes mit besonders hohem Fettsäuregehalt gewährleistet. Gleichzeitig steht dem Anwender ein sicheres und einfach zu handhabendes Aufbereitungsverfahren zur Verfügung, das neben der säulengestützten RNA-Gewinnung die sichere Kontaminationsvermeidung gewährleistet. Ebenso entfallen weitere Arbeitschritte, die dem Fachmann geläufig sind und die Qualität einer RNA-Präparation negativ beeinflussen können. Dazu gehören vor allem die Herstellung der notwendigen Puffersubstanzen, das Handling der als besonders empfindlich einzustufenden DNAse und die Vermeidung möglicher Phenolkontaminationen. Letztere inhibieren die sich anschließende Reverse Transkriptase (RT)-PCR.

Andere bislang auf dem Markt erhältliche Extraktionssysteme arbeiten auf Wasserbasis und sind daher nur wenig geeignet. Sollten diese, wie auch andere konventionelle Guanidinthiocyanat-Phenol-Extraktionen alternativ Verwendung finden, so sind die Protokolle entsprechend zu modifizieren. So wird z.B. durch mehrmaliges Passagieren der sich nach der Lyse des Probenmaterials bildenden Fettringe über die Säule oder die Fixierung des Gewebes durch Zugabe von RNAstabilisierenden Reagenzien wie z.B. RNA-Later (Fa. Qiagen, Hilden) vor dem Einsatz des Lysispuffers) eine Steigerung der RNA-Ausbeute erzielt.

Die Aufkonzentration der mRNA erfolgt alternativ auch mit polyA-gestützten Kitsystemen (z.B. Oligoresin^{®}-Kit, Fa. Qiagen, Hilden).

Beispielsweise erfolgt die Aufbereitung des Probenmaterials und die RNA-Extraktion unter folgenden Bedingungen:

1,0 ml Quiazol werden zu 50 mg zu untersuchender Probe hinzugefügt. Anschließend wird das Gemisch für den Gewebe- und Zellaufschluß in 2,0 ml Lysing Matrix D-Glastubes (Fa. Q BIOgen, Heidelberg) überführt. Die Lysing Matrix D-Glastubes sind mit im Durchmesser 1,4 mm großen Keramikkugeln bestückt und als solche Bestandteil des FastRNA^{®} Pro Green Isolationssystems (Fa. Q biogen, Heidelberg). Die Homogenisation erfolgt mit Hilfe des Fast-Prep-120 (Fa. Q Biogene, Heidelberg) mit den Einstellungen: "Geschwindigkeitsstufe 6" und "Zeitintervall 20 Sekunden" bei 4 Widerholungen. Das kurze Zeitintervall verhindert die Überhitzung der Probe und damit eine Verringerung der RNA-Ausbeute. Daran anschließend werden 200 µl Chloroform zugesetzt. Die wäßrige und die organische Phase werden durch Mikrozentrifugation bei +4°C für 15 Minuten bei 10.000 x g getrennt. Nach der Zugabe des ersten Aliquots des Waschpuffers RW1 erfolgt ein enzymatischer Abbau der DNA mit DNase (Fa. Qiagen, Hilden). Dazu werden 80 µl der DNase-Gebrauchslösung auf die Säule gegeben. Der DNase-Einsatz ist notwendig, da mit der Real Time-Technologie auch besonders geringe RNA-Konzentrationen erfaßt werden, deren Detektion durch vorhandene DANN gestört wird. Die weiteren Schritte erfolgen gemäß Protokoll des Herstellers. Das Elutionsvolumen beträgt 100 µl RNAse freies Wasser (Fa. Qiagen, Hilden). Die Elutionspassage wird zweimal durchgeführt.

### b) Reverse Transkription der RNA in cDNA

Die Umschreibung der gesamten isolierten RNA wird unter Verwendung eines Two-Step-RT-PCR-Systems mit der Multiscribe^{®}-Reverse Transkriptase und Random Hexameren durchgeführt. Dem Fachmann ist ersichtlich, daß dabei zunächst die Reverse Transkription vorgenommen wird und in einem weiteren, in separaten Reaktionsgefäßen stattfindenden, Schritt die Analyse mit der später erläuterten Real Time-PCR erfolgt. Die zur Umschreibung verwendeten Einzelkomponenten sind in Form eines Fertigkits als TaqMan^{®} Reverse Transcription Reagents (Fa. Applied Biosystems, Darmstadt) erhältlich.

Im Folgenden werden die Mastermix- und Thermocyclerkonditionen wiedergegeben. Dem Fachmann ist ersichtlich, daß die zur Transkription eingesetzte RNA-Menge (in µl) für den Anwender variabel gewählt werden kann.

### Die eingesetzten Komponenten gliedern sich wie folgt:

RNAse freies Wasser 38,5 µl, 10 x Reverse Transkriptase (RT)-Puffer: 10,0 µl, 25 mM (Magnesiumchlorid) MgCl₂: 22 µl, 2'-Desoxyribonukleosid-5'-triphosphat (dNTP): 20,0 µl, Random Hexamere: 5,0 µl, RNAse Inhibitor: 2,0 µl, Multiscribe^{®}-Reverse Transkriptase (50 U/µl): 2,5 µl**.** Als Transkriptionskontrolle wird jeweils ein RNA-Aliquot einer älteren und bereits erfolgreich umgeschriebenen RNA-Aufbereitung mitgeführt.

Die Reverse Transkription wird beispielsweise in einem GeneAmp 9600-Thermocycler der Fa. Applied Biosystems, Darmstadt, vorgenommen. Dazu werden die Proben bei 25 °C für 10 Minuten und bei 48 °C für 30 Minuten inkubiert. In Verbindung mit dem anschließenden Real Time-PCR-Analyseschritt handelt es sich um eine Two-Step-RT-PCR.

Die vergleichende Qualitätskontrolle der RNA-Präparation und des Transkriptionsprozesses erfolgt mittels UV-Spektroskopie durch Verwendung des Gerätes Beckman DU-600. Dazu werden jeweils 3 µl Probe 100fach in Wasser verdünnt. Als Standard wird gegen die RNA-Transcription Reagents (Fa. Applied Biosystems, Darmstadt) gemessen. Zur Bestimmung der Qualität der cDNA-Aufbereitung wird der Quotient aus Absorption bei 260 nm und Absorption bei 280 nm (E₂₆₀/E₂₈₀) gebildet.

Die ermittelten E₂₆₀/E₂₈₀-Werte im Bereich von 1,85-1,9 belegen dem Fachmann die qualitative Ausbeute der cDNA-Gewinnung und den sensitiven Verfahrensgang des Two-Step-Prinzips.

Dem Fachmann ist ersichtlich, daß ohne weitere erfinderische Leistungen altemative Verfahren zur Reversen Transkription verwendet werden können, ohne den Schutzumfang der Ansprüche zu verlassen. Dazu stehen eine Vielzahl von kommerziell erhältlichen Systeme zur Auswahl, beispielsweise Titan One Tube RT-PCR System, Titan One Tube RT-PCR Kit und *C. therm*. Polymerase One-Step RT-PCR System (alle Fa. Roche, Mannheim)

Darüber hinaus ist es ebenso möglich Modifikationen der Kitsysteme z.B. durch Ersetzen der Random Hexamere durch spezifische Primer oder Oligo-(dT)-Primer durchzuführen.

### c) Analyse der cDNA des GFAP-Gens in der Real Time-PCR

In der dem Fachmann zugänglichen wissenschaftlichen Literatur sind verschiedene Splicevarianten des GFAP-Gens beschrieben worden, die einen unterschiedlichen Gewebetropismus und damit auch eine unterschiedliche Eignung für den selektiven ZNS-Nachweis aufweisen. Es ist allgemein zu berücksichtigen, dass eine wissenschaftliche Bewertung hinsichtlich des Vorkommens der GFAP-Varianten bei den für die verschiedenen Formen der TSE-Erkrankung relevanten Tierspezies nicht existiert. Umfassendes Sequenzmaterial liegt bislang nur für den Menschen und die Tierarten Maus und Ratte vor. Die vorliegenden Erfindung orientiert sich an der für die Ratte beschriebenen delta-Form des GFAP-Gens, die aus 9 Exons besteht und, das bestätigen die eigenen Ergebnisse mit dem sehr sensitiven Real Time-PCR-Verfahren, nahezu ausschließlich in ZNS- und Rückenmark vorherrscht. Alternativ ist die Verwendung weitere Splicevarianten, etwa der epsi-Ion-Ausprägung möglich.

Voraussetzung für die Etablierung des Verfahrens für den tierartspezifischen Nachweis von ZNS-Material aus Schaf und Ziege ist die Bereitstellung der Nukleotidsequenzen des GFAP-Gens von Schaf und Ziege und ein vergleichendes Alignment für die Tierarten Schaf, Ziege, Schwein und Rind, da beides im Stand der Technik noch nicht bekannt ist.

Zwar gibt es Informationen auf Aminosäurebasis, diese sind aber Tierartunspezifisch und können zur Durchführung des erfindungsgemäßen Verfahrens nicht verwendet werden. Daher ist es zunächst wichtig, umfangreiches Sequenzmaterial für die Tierarten Rind, Schaf, Ziege und Schwein bereitzustellen und mit den bislang über die Genbank zugänglichen Sequenzfragmenten zu vergleichen.

Dies wird wie folgt durchgeführt:
- Herstellung von Referenz-DNA der Tierarten Rind, Schaf, Ziege und Schwein
- Sequenzierung der GFAP-Gene der Tierarten Rind, Schaf, Ziege und Schwein
- Analyse der GFAP-Sequenzen mittels Genstrukturbestimmung und Alignment

Zur Herstellung von Referenz-DNA der Tierarten Rind, Schaf, Ziege und Schwein wird als Ausgangsmaterial genomische DNA aus quergestreifter Muskulatur gewonnen. Die DNA-Extraktion erfolgt nach bekannten DNA-Extraktionsverfahren beispielsweise unter Verwendung des Dneasy^{®} Tissue Kits (Fa. Qiagen, Hilden). Das Verfahren wird nach Herstellerangaben durchgeführt.

Das Vorliegen einer intakten DNA-Präparation wird durch PCR-Überprüfung mittels universeller Primer beispielsweise für das *Cytochrome B*-Gen, kontrolliert. Die Lagerung der Referenz-DNA erfolgt bei -20°C.

Für die Sequenzierung der GFAP-Gene der Wiederkäuer-Tierarten Rind, Schaf, Ziege und der Tierart Schwein werden, nach dem Fachmann bekannten Verfahren, geeignete Sequenzierungsprimer verwendet und die Sequenzierung beispielsweise mit dem MegaBACE 1000 DNA Sequencing System (Fa. Amersham Pharmacia Biotech) nach Herstellervorgaben durchgeführt.

Die Bearbeitung der Rohdaten erfolgt via Chromas (School of Biomolecular and Biomedical Science and Technology, Griffith University, Brisbane, Queensland, Australia) und anschließender Datenkonvertierung mit dem Softwarepaket DNA-Star (Fa. Lasergene^{®}). Zur Festlegung der Exon- und Intron-Grenzen wird zusätzlich zur cDNA-Analyse und den genannten Computerprogrammen auf verschiedene Intemetdatenbanken (beispielsweise BLAST der NCBI-Gendatenbank) zurückgegriffen.

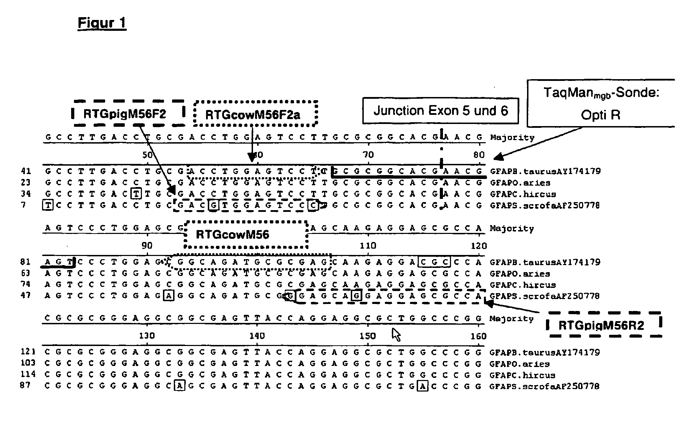

Der Vergleich der Sequenzen in der GFAP-Sequenzanalyse ist in Figur 1 schematisch dargestellt. Dazu zeigt Figur 1 das Alignment der GFAP-Untereinheiten der Tierarten Rind (*Bos taurus*), Schaf (*Ovis aries*), Ziege (*Caprae hircus*) und Schwein (*Sus scrofa*) und die Lokalisation der verwendeten Primer und der TaqMan_{mgb}-Sonde. Der Ausschnitt zeigt die für das Primer und Sondendesign gewählten Bereiche der aneinander grenzenden Exons 5 und 6. Dabei ist grundsätzlich die hohe Sequenzhomologie innerhalb der Wiederkäuer Rind, Schaf und Ziege im Gegensatz zur Tierart Schwein deutlich erkennbar.

Figur 1 zeigt a) die Primer- und Sondenkombination zur Detektion der Tierarten Rind, Schaf und Ziege:

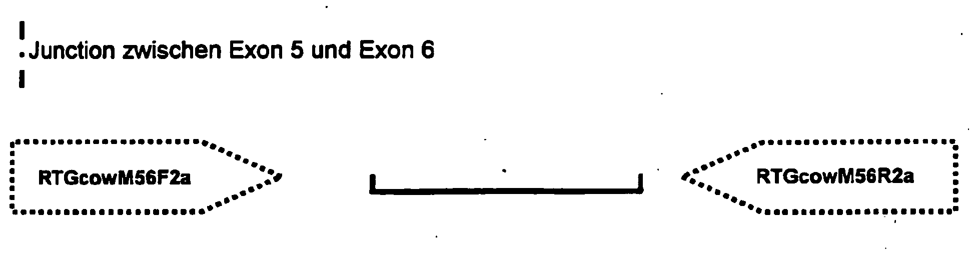

### b) die Primer- und Sondenkombination zur Detektion der Tierart Schwein:

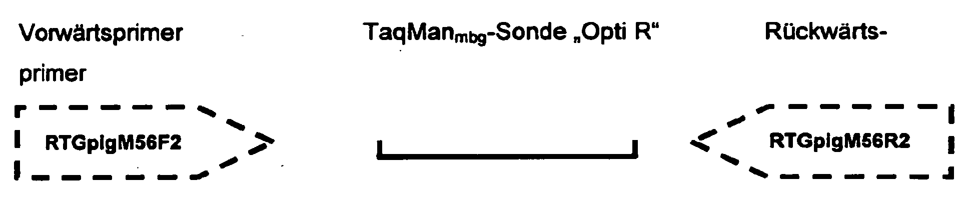

Die GFAP-Gensequenz des Rindes (*Bos taurus*, Guertler et al., 2003) ist unter der Genbankzugriffsnummer AY 174179, die GFAP-Gensequenz des Schweines (*Sus scrofa*, Yu et al., 2001) unter AF 250778 in der NCBI-Genbank einsehbar.
Aus dem Sequenzvergleich wird ersichtlich, daß die gewählten Oligonukleotide entsprechend den dem Fachmann geläufigen Regeln zur Auswahl von PCR-Primem im untersuchten Sequenzabschnitt optimal positioniert sind, um eine selektive Erfassung der GFAP-Gensequenzen der Tierarten Rind, Schaf und Ziege ( 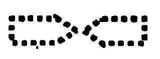 ) zu gewährleisten.
Analog dazu wurde ein weiteres Primerpaar so gewählt, daß selektiv die GFAP-Gensequenz der Tierart Schwein 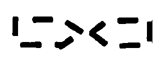 amplifiziert wird.

Das Design der die Exon-Exon-Grenze überspannenden TaqMan_{mgb}-Sonde ( 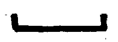 ) entspricht den Vorgaben, d.h., dass die Junction genau in die Mitte der Sonde gelegt wird, Temperatur und G:C-Verhältnis optimal auf die entwickelten Primer abgestimmt sind. Die Positionierung der Sonde gewährleistet die selektive Amplifikation von cDNA. Das Amplifikatgrößen (für Rind, Schaf und Ziege circa 80 Basenpaare, für Schwein circa 60 Basenpaare) sind optimal für die Erfordernisse der Real Tirne-Technologie, für die nach wissenschaftlichen und technischen Statuten eine Produktgröße von 50 bis 150 Basenpaaren gefordert wird. Die geringe Produktgröße ist eine wesentliche Voraussetzung für die Gehwährleistung der hohe Sensitivität und Spezifität, das schließt zudem die positive Nachweisführung auch nach 2-jähriger Lagerungsdauer von hitzebehandelten Proben mit ein.

Aufgrund des Datenmaterials in diesem Umfang und unter Einbeziehung der genannten Spezies ist es erstmals möglich, die Tierartspezifität des GFAP-Gens darzustellen. Dabei wird vorzugsweise der Genabschnitt Exon 5 und Exon 6 des GFAP-Gens verwendet, um Spezies-spezifische Primer zu generieren. Ebenso wird eine die Grenze zwischen Exon 5 und Exon 6 überspannende TaqMan_{mgb}-Sonde entwickelt.
Mit der Bereitstellung der GFAP-Nukleotidsequenzen für die Tierarten Schaf und Ziege sind die Voraussetzungen für die selektive tierartspezifische Erfassung von cDNA der Wiederkäuer-Tierarten Rind, Schaf, Ziege auf der einen und der Tierart Schwein auf der anderen Seite vorhanden.

Alternativ kann können zur Durchführung des erfindungsgemäßen Verfahrens auch andere Exons beispielsweise Exon 1, 2, 3, 4 und 7, 8 und 9 wie auch die Regionen der unterschiedlichen Splicevarianten, z.B. Exon 7a der epsilon-Ausprägung des GFAP-Gens, verwendet werden, die Herstellung der entsprechenden Primer ist dem Fachmann geläufig und ohne weitere erfinderische Leistungen leicht möglich. Ebenfalls wird das erfindungsgemäße Verfahren auf den Nachweis von ZNS-Material von Wiederkäuer-Tierarten generell, auch auf die Wildwiederkäuer angewandt, da diese eine ähnliche Nukleotidsequenz des GFAP-Gens aufweisen. Bei entsprechender Kenntnis der Nukleotidsequenz des GFAP-Gens von anderen Wirbeltieren, beispielsweise der Equiden, wie Pferd und Esel oder Geflügel, beispielsweise Huhn, Pute und Ente, wie auch der weiteren Säugetiere, wie Nerz oder Hund und Katze, ist die Herstellung der entsprechenden Primer und die Durchführung des erfindungsgemäßen Verfahren dem Fachmann ersichtlich.

In einem bevorzugten Ausführungsbeispiel wird das Verfahren so durchgeführt, daß zum einen der spezifische Nachweis für ZNS-Material der Wiederkäuer-Tierarten Rind, Schaf und Ziege und zum anderen für die Tierart Schwein erfolgt.

Auf Basis der Nukleotidsequenzen des GFAP-Gens der Wiederkäuer-Tierarten Rind, Schaf und Ziege werden Primer- und TaqMan_{mgb}-Sondensequenzen vorzugsweise unter Nutzung von Exon 5 und Exon 6 entwickelt.
Das erfindungsgemäße Verfahren wird dadurch so durchgeführt, daß der spezifische Nachweis für ZNS-Material der Tierarten Rind, Schaf und Ziege erfolgt.

Die Sequenzen der erfindungsgemäßen Primer zur Analyse der cDNA in der Real Time- PCR und die der TaqMan_{mgb}-Sonde setzen sich aus folgenden Nukleinsäuresequenzen zusammen:

| **Nomenklatur der** | | **Nukleotidsequenz** | **Basenanzahl** |
|---|---|---|---|
| **Vorwärtsprimer** | RTGcowM56F2a | 5'-ACC TGC GAC CTG GAG TCC T-3' | 19 |
| **Rückwärtsprimer** | RTGcowM56R2a | 5'-CTC GCG CAT CTG CCG-3' | 15 |
| **TaqMan_{mgb}-Sonde** | OptiR | 6-FAM-ACT CGT TCG TGC CGC GC-MGB | 17 |

Auf Basis der Nukleotidsequenzen des GFAP-Gens der Tierarten Schwein werden Primer- und TaqMan_{mgb}-Sondensequenzen vorzugsweise unter Nutzung von Exon 5 und Exon 6entwickelt.
Das erfindungsgemäße Verfahren wird dadurch so durchgeführt, daß der spezifische Nachweis für ZNS-Material der Tierart Schwein erfolgt.

Die Sequenzen der erfindungsgemäßen Primer zur Analyse der cDNA in der Real Time- PCR und die der TaqMan_{mgb}-Sonde setzen sich aus folgenden Nukleinsäuresequenzen zusammen:

| **Nomenklatur der** | | **Nukleotidsequenz** | **Basenanzahl** |
|---|---|---|---|
| **Vorwärtsprimer** | RTGpigM56F2 | 5'-GAC CTG CGA CGT GGA GTC CC-3' | 20 |
| **Rückwärtsprimer** | RTGpigM56R2 | 5'-TGG CGC TCC TCC TGC TCC -3' | 18 |
| **TaqMan_{mgb}-Sonde** | OptiR | 6-FAM-ACT CGT TCG TGC CGC GC-MGB | 17 |

### Erläuterungen zur Nomenklatur für die Primer und Sondenkombinationen:

TaqMan^{®} ist ein eingetragenes Warenzeichen der Fa. Hoffman-La Roche.

Die Bezeichnung mgb/MGB steht für minor groove binder und bezeichnet eine dem Fachmann geläufige Methode des Sondendesigns mit der eine höher Bindungsaffinität zur Zielregion und die Auswahl von Sonden mit einer kürzeren Basensequenz ermöglicht wird. Dieses methodische Vorgehen hat sich für die dargestellte Region als besonders vorteilhaft erwiesen.

FAM bezeichnet eine dem Fachmann geläufige, für die Fluoreszenzfarbstoffmarkierung von Sonden gewählte Substanz, die alternativ auch als Fluorescein bezeichnet wird. Der Einsatz weiterer Flurophore entspricht dem technischen Standard und ist dem Fachmann geläufig.

Die erfindungsgemäße TaqMan_{mgb}-Sonde wird jeweils alternativ mit einem der beiden Primerpaare eingesetzt. Der mittels dieser Primer- und Sondenkombination zur Verfügung stehende Real Time-PCR-Assay ist damit besonders zur tierartspezifischen und quantitativen Erfassung von ZNS-Gewebe der Tierarten Rind, Schaf und Ziege bzw. Schwein geeignet. Als Analysegerät wird das ABI 7000 Sequence Detection System (Fa. Applied Biosystems, Darmstadt) genutzt.

Es wird vorgeschlagen, die erfindungsgemäßen Primer alternativ mit einer Markierung (Fluoreszenz, Radioaktivität und ähnliches) zu versehen und die Analyse durch Autoradiografie oder Chemilumineszenz durchzuführen.

Die erfindungsgemäßen Primer sind Nukleinsäuremoleküle, die an das GFAP-Gen binden bzw. mit ihm hybridisierenden. Abweichend von den angegebenen Nukleotidsequenzen umfasst dies deren Fragmente, Derivate und allelische Varianten. Derivat bedeutet in diesem Zusammenhang, daß die Sequenzen dieser Primer sich an einer oder mehreren Positionen unterscheiden und einen hohen Grad an Homologie. Homologie meint dabei eine Sequenzidentität von mindestens 40 %, insbesondere eine Identität von mindestens 60 %, vorzugsweise über 80 % und besonders bevorzugt über 90 %. Die Abweichungen zu den oben beschriebenen Nukleinsäuremolekülen können dabei durch Deletion, Substitution, und/oder Insertion entstanden sein.

Für den dem Fachmann geläufigen Verfahrensschritt der relativen Quantifizierung, wird cDNA aus dem ZNS-Gewebe- enthaltendem Probenmaterial, das zuvor nach dem beschriebenen Prozeß aufbereitet wird, als dekadische Verdünnungsreihe in der jeweiligen Real Time-PCR eingesetzt.

Dabei wird die Konzentration der gewonnenen cDNA via UV-Spektroskopie bestimmt. Anschließend wird im Rahmen der Konfiguration des verwendeten Real Time-Cyclers die dekadische Verdünnungsreihe als Standard definiert und die spektroskopisch ermittelten Werte manuell als Berechnungsgrundlage in die Bedienungssoftware des Thermocyclers einzugeben. Der cDNA-Gehalt der zu untersuchenden Proben wird als Relation zu den im gleichen PCR-Lauf mitgeführten Standards ermittelt.

Alternativ besteht die Möglichkeit, die diagnostische Empfindlichkeit durch das Verwenden einer absoluten Quantifizierung mittels sog. Housekeeping-Gene weiter zu steigern.

Folgende Mastermixkonditionen (50 µl-Ansatz) werden bevorzugt eingesetzt:

| Komponenten für den Mastermix | Versuchsansatz 1x in µl je 50 µl |
|---|---|
| TaqMan Universal PCR Master Mix (2x) | 25,0 |
| MgCl₂ | 0 |
| Primer 1 | 1,5 |
| Primer 2 | 1,5 |
| TaqMan-Sonde | 2,5 |
| Aqua dest. | 14,5 |
| Template-DNA | 5,0 |
| Total volume | 50 µl |

Die verwendeten Reagenzien sind als Komplettformulierung im TaqMan-Universal Mastermix (Fa. Applied Biosystems, Darmstadt) enthalten.

Die Thermocylclerkonditionen werden wie folgt gewählt:

Initale UNG-Inkubation bei 50 °C für 2 min, Aktivierung der AmpliTaq Gold Polymerase (Fa. Applied Biosystems, Darmstadt) bei 95 °C. Als Analysezeitraum werden 40 Zyklen festgelegt, die eine Denaturierung bei 95 °C für 15 S und eine Annealingphase bei 60 °C für 1 Minute umfassen. Generell erfolgt jede Messung im Duplikatansatz. Die Datenauswertung, bestehend aus der Festlegung des Schwellenwertzyklusses (Cₜ-Wert) und der Bestimmung des quantitativen GFAP-Gehaltes [Einheit: ng/µl] in der untersuchten Probe wird nach den dem Fachmann geläufigen Methoden vorgenommen.

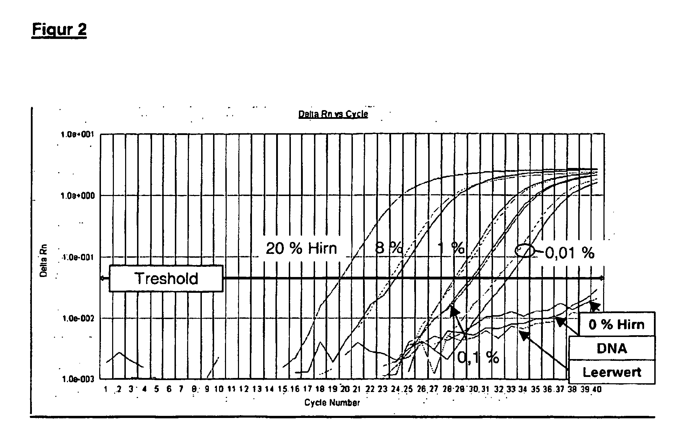

Die abschließende Auswertung der Ergebnisse erfolgt nach der dem Fachmann geläufigen Vorgehensweise, die exemplarisch in der Figur 2 für die Untersuchung roher und hitzebehandelter Fleischerzeugnisse dargestellt ist. Figur 2 zeigt die Darstellung der Nachweisgrenze des Real Time-PCR-Systems.

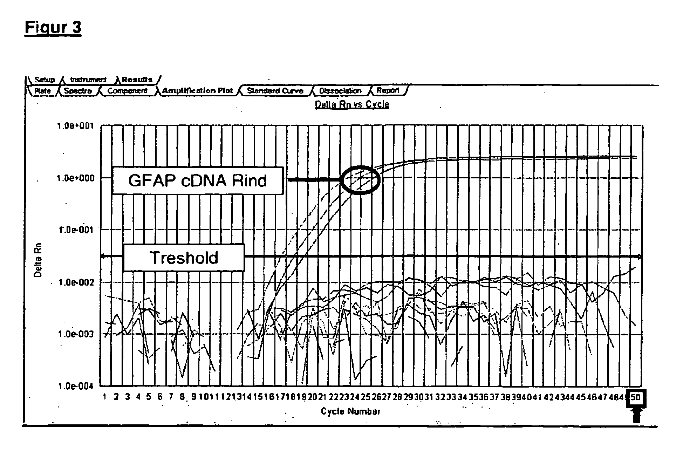

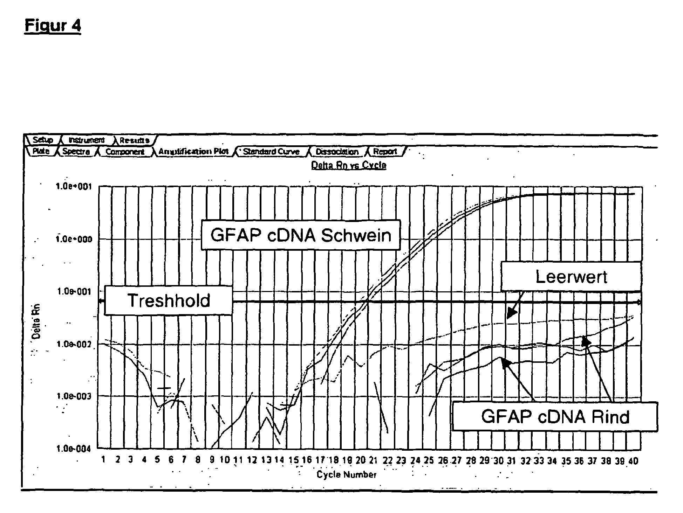

Figur 3 und 4 zeigen die Tierartspezifische Nachweisführung mit dem entwickelten Real Time-PCR-System. Figur 3 zeigt die selektive Erfassung von GFAP cDNA der Tierart Rind, bei den nicht detektierten Tierarten handelt es sich um Schwein, Pute. Huhn und Ente. Der dargestellte Fluoreszenzsignalverlauf gilt entsprechend für die Tierarten Schaf und Ziege. Figur 4 zeigt die selektive Erfassung von GFAP cDNA der Tierart Schwein. Der Untersucher erhält als Ergebnis den prozentualen und lebensmittelrechtlich relevanten ZNS-Gehalt des analysierten Fleischerzeugnisses. Dieser wird wie folgt ermittelt:
1. Dem Fachmann ist die Darstellungsweise der Signaldetektion als "Amplification plot" geläufig. Die Fluoreszenzsignalerfassung, bestimmt anhand des Schwellenwertzyklusses (Cₜ-Wertes), die äquivalent zur Bildung des erwünschten Amplifikates ist, setzt um so früher ein, je höher die GFAP-Ausgangskonzentration in der untersuchten Probe ist.
   Wie exemplarisch dargestellt, entspricht ein Schwellenwertzyklus von Cₜ-Wertes von 32,4 bzw. 33,1 einem ZNS-Gehalt von 0,01. % Gehimgewebe in hitzebehandelten Fleischerzeugnissen z.B. Halbkonserven, Dreiviertel- und Vollkonserven, ebenso wie für rohe Fleischerzeugnisse. Dabei ist dem Fachmann bekannt, dass es sich insbesondere bei Vollkonserven, die im Rahmen der Herstellung einen Erhitzungsprozeß bei 121°C durchlaufen, um Erzeugnisse handelt, bei denen ein hohes Maß an Schädigung der zu detektierenden mRNA zu erwarten ist. Die äußerst zuverlässige Nachweisrate des entwickelten Verfahrens auch bei dieser Produktgruppe ist als eindeutiger Vorteil im Vergleich zu den bislang existierenden Methoden anzusehen. Die untersuchten Proben werden jeweils im Doppelansatz analysiert. Dabei ist dem Fachmann ersichtlich, daß im niedrig konzentrierten mRNA-Bereich (hier: 0,01 %) innerhalb eines PCR-Ansatzes Cₜ-Wertschwankungen von 0,2 bis 1,0 als verfahrensbedingt zu werten sind.
   Anhand des "Amplification plots" ist deutlich zu erkennen, dass keine "falsch-positiven" Reaktionen innerhalb des 0-Wertes, also der Probe, der kein Gehirn zugesetzt wird, erfolgen. Die selektive cDNA-Erfassung wird anhand der mitgeführten DNA-Aufbereitung ersichtlich. In dieser Probe findet keine Reaktion statt. Damit wird die Zuverlässigkeit des Real Time-PCR-Systems insbesondere unter Berücksichtigung der diagnostischen Qualitätssicherung sichergestellt. Mit der erfindungsgemäßen Methodenkaskade ist es erstmals möglich, auch geringste Schwankungen bzw. Verringerungen des GFAP-Gehaltes, wie sie etwa durch die extreme Hitzebehandlung des Lebensmittels bei der Konservenherstellung entstehen können, nachzuweisen.
2. Weiterhin werden die Daten in Form der dem Fachmann geläufigen tabellarischen Übersicht der Untersuchungsergebnisse, die bei dem exemplarisch verwendeten Real Time-Cycler Abi PRISM^{®} 7000 Sequence Detection System als "Analysis Report" zusammengefaßt werden und je nach verwendetem Gerätetyp analoge Bezeichnungen tragen, ausgegeben. Hier werden neben den Cₜ-Werten auch die quantitativen Relationen im Vergleich zur mitgeführten Verdünnungsreihe mit der Einheit Nanogramm pro PCR-Reaktion [ng/PCR-Reaktion] aufgeführt, die folgende Interpretation gewährleisten: Der Gehalt an GFAP-cDNA in ng/PCR-Ansatz in der untersuchten Probe entspricht dem einer Referenzprobe mit einem festgelegten prozentualen Anteil an Himzusatz. Dem Fachmann ist ersichtlich, dass alternativ mit der Durchführung einer absoluten Quantifizierung der GFAP-Gehalt als Anzahl der in der Probe detektierten Kopienzahl des Gens angegeben werden kann.

Es ist hervorzuheben, daß die elektronische Datenerfassung und Dokumentation den Vorgaben im Rahmen der diagnostischen Qualitätssicherung für die moderne Laborpraxis entsprechen.

Eine Ausführungsform der Erfindung betrifft einen einfach zu handhabenden Kit zum tierartspezifischen und quantitativen Nachweis von ZNS-Gewebe in Fleisch und Fleischerzeugnissen, mindestens enthaltend
1. Materialien zur tierartspezifischen und quantitativen Analyse der GFAP cDNA. Materialien zum Nachweis der GFAP cDNA der Tierarten Rind, Schaf und Ziege für Real Time-PCR TaqMan sind: Universal PCR Master, MgCl₂, Primer RTGcowM56F2a 5'-ACC TGC GAC CTG GAG TCC T-3', Primer RTGcowM56R2a 5'-CTC GCG CAT CTG CCG-3' und TaqMan_{mgb}-Sonde OptiR 6-FAM-ACT CGT TCG TGC CGC GC-MGB.
Materialien zum Nachweis der GFAP cDNA der Tierart Schwein sind: Universal PCR Master, MgCl₂, Primer RTGpigM56F2 5'-GAC CTG CGA CGT GGA GTC CC-3', Primer RTGpigM56R2 5'-TGG CGC TCC TCC TGC TCC -3' und TaqMan_{mgb}-Sonde OptiR 6-FAM-ACT CGT TCG TGC CGC GC-MGB.

### Alternativ enthält der Testkit zusätzlich

11. Materialien zur RNA-Extraktion sowie geeignete Reaktionspuffer und/oder III. Materialien zur Reversen Transkription der extrahierten GFAP mRNA Beispielsweise RNAse freies Wasser, Reverse Transkriptase (RT)-Puffer, MgCl₂, 2'-Desoxyribonukleosid-5'-triphosphat (dNTP, Random Hexamere, RNAse Inhibitor, Reverse Transkriptase. Alternativ enthält der Testkit eine Transkriptionskontrolle in Form einer GFAP mRNA zur Überwachung einer erfolgreichen Umschreibungsprozesses der isolierten GFAP mRNA in cDNA.

Darüberhinaus enthält der Testkit alternativ eine Positivkontrolle in Form der GFAP cDNA der Tierart Rind bzw. Schwein und eine Negativkontrolle in Form der GFAP cDNA der Tierart Rind bzw. Schwein.
Bevorzugt enthält der Testkit eine Interne Amplifikationskontrolle, sie dient zur Überwachung des korrekten PCR-Analyseablaufes und schließt "falsch-negative"-Ergebnisse aus.
Bevorzugt enthält der Testkit Referenzproben zur Quantifizierung der untersuchten Testproben. Dem Fachmann ist geläufig, dass dies einerseits die Vorlage der bereits beschriebenen Verdünnungsreihe ist, sowie die Zugabe verschiedener Proben mit definiertem ZNS-Gehalt. Alternativ ist die Referenzprobe ein Referenzgens zur absoluten Quantifizierung.

Der Testkit schließt die Formulierung als Einzeldetektionssystem ebenso ein, wie die Auslegung als Multiplex-PCR.

Auf Grund der Lehre der vorliegenden Erfindung sowie auf Grund des allgemeinen Fachwissens in diesem technischen Gebiet ist dem Hersteller des erfindungsgemäßen Kits bekannt, wie er die einzelne Komponenten des Kits, z.B. die Puffer herstellt, formuliert und lagert.

## Patentansprüche

1. Verfahren zum tierartspezifischen und quantitativen Nachweis von ZNS-Gewebe in Fleisch und Fleischerzeugnissen,
**gekennzeichnet durch** die Schritte
a) Aufbereitung des Probenmaterials und RNA-Extraktion
b) Reverse Transkription der RNA in cDNA
c) Analyse der cONA des GFAP-Gens in der Real Time-PCR, wobei die folgenden Primer verwendet werden,
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAG CTG CGA CGT GGA GTC CC-3' |
| RTGpigM56R2: | 5'-TGG CGC TCC TCC TGC TCC-3'. |

2. Verfahren gemäß Anspruch 1, **dadurch gekennzeichnet, dass** es spezifisch für die Tierarten Rind, Schaf, Ziege und Schwein ist.

3. Verfahren gemäß Anspruch 1, **dadurch gekennzeichnet, dass** die Aufbereitung des Probenmaterials durch Homogenisation erfolgt, vorzugsweise aus einer Kombination vertikaler Rotations- und horizontaler Auf-und Ab-Bewegungen.

4. Verfahren gemäß der vorhergehenden Ansprüche, **dadurch gekennzeichnet, dass** die RNA-Extraktion durch Lyse und Extraktion auf Phenolbasis erfolgt, so dass RNA auch aus Matrizes mit besonders hohem Fettsäuregehalt extrahiert wird.

5. Verfahren gemäß der vorhergehenden Ansprüche, **dadurch gekennzeichnet, dass** die Real Time-PCR für die Tierarten Rind, Schaf und Ziege durchgeführt wird mit
| | |
|---|---|
| Primer RTGcowM56F2a | 5'-ACC TGC GAC CTG GAG TCC T-3' |
| Primer RTGcowM56R2a | 5'-CTC GCG CAT CTG CCG-3' |
| TaqMan_{mgb}-Sonde OptiR | 6-FAM-ACT CGT TCG TGC CGC GC-MGB. |

6. Verfahren gemäß Anspruch 5, **dadurch gekennzeichnet, dass** Primer RTGcowM56F2a oder Primer RTGcowM56R2a mit der TaqMan_{mgb}-Sonde OptiR verwendet wird.

7. Verfahren gemäß der vorhergehenden Ansprüche, **dadurch gekennzeichnet, dass** die Real Time-PCR für die Tierart Schwein mit folgenden Primem durchgeführt wird
| | |
|---|---|
| Primer RTGpigM56F2 | 5'-GAC CTG CGA CGT GGA GTC CC-3' |
| Primer RTGpigM56R2 | 5'-TGG CGC TCC TCC TGC TCC-3' |
| TaqMan_{mgb}-Sonde OptiR | 6-FAM-ACT CGT TCG TGC CGC GC-MGB. |

8. Verfahren gemäß Anspruch 7, **dadurch gekennzeichnet, dass** Primer RTG RTGpigM56F2 oder Primer RTGpigM56R2 mit derTaqMan_{mgb}-Sonde OptiR verwendet wird.

9. Verfahren gemäß der vorhergehenden Ansprüche, **dadurch gekennzeichnet, dass** es In Hitze-behandeltem Fleisch und Fleischerzeugnisse durchgeführt wird.

10. Verwendung des Verfahrens gemäß Anspruch 1 zum tierartspezifischen und quantitativen Nachweis von ZNS-Gewebe in Fleisch und Fleischerzeugnissen.

11. Testkit zum tierartspezifischen und quantitativen Nachweis von ZNS-Gewebe in Fleisch und Fleischerzeugnissen, mindestens enthaltend die Primer
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3' |
zur tierartspezifischen und quantitativen Analyse der GFAP cDNA.

12. Testkit zum tierartspezifischen und quantitativen Nachweis von ZNS-Gewebe in Fleisch und Fleischerzeugnissen gemäß Anspruch 11, enthaltend Materialien zur RNA-Extraktion sowie geeignete Reaktionspuffer und/oder Materialien zur Reversen Transkription der extrahierten GFAP mRNA.

13. Testkit zum tierartspezifischen und quantitativen Nachweis von ZNS-Gewebe in Fleisch und Fleischerzeugnissen, gemäß Anspruch 12, **dadurch gekennzeichnet, dass** die Materialien zur RNA-Extraktion RNAse freies Wasser, Reverse Transkriptase (RT) -Puffer. MgCl₂, 2'-Desoxyribonukleosid-5'-triphosphat (dNTP), Random Hexamere, RNAse Inhibitor und Reverse Transkriptase sind.

14. Testkit zum tierartspezifischen und quantitativen Nachweis von ZNS-Gewebe in Fleisch und Fleischerzeugnissen, gemäß Anspruch 11 bis 13, **dadurch gekennzeichnet, dass** eine Transkriptionskontrolle in Form einer GFAP mRNA zur Überwachung eines erfolgreichen Umschreibungsprozesses der isolierten GFAP mRNA in cDNA enthalten ist.

15. Testkit zum tierartspezifischen und quantitativen Nachweis von ZNS-Gewebe in Fleisch und Fleischerzeugnissen gemäß Anspruch 11 bis 14, **dadurch gekennzeichnet, dass** die Materialien zur Reversen Transkription der extrahierten GFAP mRNA zum Nachweis der Tierarten Rind, Schaf und Ziege Universal PCR Master, MgCl₂, Primer RTGcowM56F2a 5'-ACC TGC GAC CTG GAG TCC T-3', Primer RTGcowM56R2a 5'-CTC GCG CAT CTG CCG-3' und TaqMan_{mgb}-Sonde OptiR 6-FAM-ACT CGT TCG TGC CGC GC-MGB sind.

16. Testkit zum tierartspezifischen und quantitativen Nachweis von ZNS-Gewebe in Fleisch und Fleischerzeugnissen, gemäß Anspruch 11-15; **dadurch gekennzeichnet, dass** die Materialien zur Reversen Transkription der extrahierten GFAP mRNA zum Nachweis der Tierart Schwein Universal PCR. Master, MgCl₂, Primer RTGpigM56F2 5'-GAC CTG CGA CGT GGA GTC CC-3', Primer RTGpigM56R2 5'-TGG CGC TCC TCC TGC TCC-3' und TaqMan_{mgb}- Sonde OptiR 6-FAM-ACT CGT TCG TGC CGC GC-MGB sind.

17. Testkit zum tierartspezifischen und quantitativen Nachweis von ZNS-Gewebe in Fleisch und Fleischerzeugnissen, gemäß Anspruch 11-16, **dadurch gekennzeichnet, dass** er eine Positivkontrolle in Form der GFAP cDNA der Tierart Rind bzw. Schwein und eine Negativkontrolle in Form der GFAP cDNA der Tierart Rind bzw. Schwein, eine interne Amplifikationskontrolle, sowie Referenzproben zur Quantifizierung der untersuchten Testproben enthält.

18. Testkit zum tierartspezifischen und quantitativen Nachweis von ZNS-Gewebe in Fleisch und Fleischerzeugnissen, gemäß Anspruch 11-17, **dadurch gekennzeichnet, dass** die Referenzproben, Verdünnungsreihen, Proben mit definiertem ZNS-Gehalt und/oder ein Referenzgen sind.

## Claims

1. Procedure for the species-specific and quantitative detection of CNS tissue in meat and meat products,
**characterized by** the steps:
a) Preparation of the test material and NA extraction
b) Reverse transcription of the RNA to cDNA
c) Analysis of the cDNA of the GFAP gene in real time PCR by using the following primers,
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3'. |

2. Procedure according to Claim 1, **characterized in that** it is specific for the cow, sheep, goat and pig species.

3. Procedure according to Claim 1, **characterized in that** the preparation of the test material is done by homogenization, preferably of a combination of vertical rotation and horizontal up-and-down movements.

4. Procedure according to the above Claims, **characterized in that** the RNA extraction is done by lysis and extraction on a phenol basis so that RNA is extracted also from matrices of an extremely high fatty acid concentration.

5. Procedure according to the above Claims, **characterized in that** the real time PCR for the cow, sheep and goat species is done with
| | |
|---|---|
| Primer RTGcowM56F2a | 5'-ACC TGC GAC CTG GAG TCC T-3' |
| Primer RTGcowM56R2a | 5'-CTC GCG CAT CTG CCG-3' |
| TaqMan_{mgb}-probe OptiR | 6-FAM-ACT CGT TCG TGC CGC GC-MGB. |

6. Procedure according to Claim 5, **characterized in that** the primer RTGcowM56F2a or the primer RTGcowM56R2a is used with the TaqMan_{mgb}probe OptiR.

7. Procedure according to the above Claims, **characterized in that** the real time PCR for the pig species is done with the following primers
| | |
|---|---|
| Primer RTGpigM56F2 | 5'-GAC CTG CGA CGT GGA GTC CC-3' |
| Primer RTGpigM56R2 | 5'-TGG CGC TCC TCC TGC TCC-3' |
| TaqMan_{mgb}-probe OptiR | 6-FAM-ACT CGT TCG TGC CGC GC-MGB. |

8. Procedure according to Claim 7, **characterized in that** the primer RTGpigM56F2 or the primer TRGpigM56R2 is used with the TaqMan_{mgb}-probe OptiR.

9. Procedure according to the above Claims, **characterized in that** it is done in heat-treated meat and meat products.

10. Use of the procedure according to Claim 1 for the species-specific and quantitative detection of CNS-tissue in meat and meat products.

11. Test kit for the species-specific and quantitative detection of CNS-tissue in meat and meat products, containing at least the primers
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3' |
for the species-specific and quantitative analysis of the GFAP cDNA.

12. Test kit for the species-specific and quantitative detection of CNS-tissue in meat and meat products according to Claim 11, containing materials for RNA extraction as well as suitable reaction buffers and/or materials for the reverse transcription of the extracted GFAP mRNA.

13. Test kit for the species-specific and quantitative detection of CNS-tissue in meat and meat products according to Claim 12, **characterized in that** the materials for RNA extraction are free water, reverse transcriptase (RT)-buffers, MgCl₂, 2'-desoxyribo-nudeoside-'5-triphosphate (dNTP), random hexamers, RNAse inhibitors and reverse transcriptase.

14. Test kit for the species-specific and quantitative detection of CNS-tissue in meat and meat products according to the Claims 11 to 13, **characterized in that** it contains a transcription control in form of a GFAP mRNA to survey a successful transcription process of the isolated GFAP mRNA to cDNA.

15. Test kit for the species-specific and quantitative detection of CNS-tissue in meat and meat products according to the Claims 11 to 14, **characterized in that** the materials for the reverse transcription of the extracted GFAP mRNA for the detection of the cow, sheep and goat species are Universal PCR Master, MgCl₂, primer RTGcowM56F2a 5'-ACC TGC GAC CTG GAG TCC T-3', primer RTGcowM56R2a 5'-CTC GCG CAT CTG CCG-3', and TaqMan_{mgb}-probe OptiR 6-FAM-ACT CGT TCG TGC CGC GC-MGB.

16. Test kit for the species-specific and quantitative detection of CNS-tissue in meat and meat products according to the Claims 11 - 15, **characterized in that** the materials for the reverse transcription of the extracted GFAP mDNA for the detection of the pig species are Universal PCR Master, MgCl₂, primer RTGpigM56F2 5'-GAC CTG CGA CGT GGA GTC CC-3', primer RTGpigM56R2 5'-TGG CGC TCC TCC TGC TCC-3', and TaqMan_{mgb}-probe OptiR 6-FAM-ACT CGT TCG TGC CGC GC-MGB.

17. Test kit for the species-specific and quantitative detection of CNS-tissue in meat and meat products according to the Claims 11 - 16, **characterized in that** it contains a positive control in form of the GFAP cDNA of the cow respectively the pig species, and a negative control in form of the GFAP cDNA of the cow respectively the pig species, an internal amplification control, as well as reference samples for the quantification of the examined test samples.

18. Test kit for the species-specific and quantitative detection of CNS-tissue in meat and meat products according to the Claims 11 - 17, **characterized in that** the reference samples are dilution series, samples of a defined CNS-contents, and/or a reference gene.

## Revendications

1. Procédure pour l'identification quantitative et spécifique d'espèce d'animal du tissue du système nerveux central dans la viande et des produits de viande, **caractérisée par** les pas:
a) Préparation de l'essai et de l'extraction de l'ARN
b) Transcription inverse de l'ARN en cADN
c) Analyse du cADN du gène GFAP dans la réaction en chaîne polymérase en temps réel en utilisant les amorces suivantes,
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3'. |

2. Procédure selon la Revendication1, **caractérisée en ce qu'**il est spécifique pour les espèces d'animaux de boeuf, mouton, chèvre, et cochon.

3. Procédure selon la Revendication1, **caractérisée en ce que** la préparation du matériel d'essai est faite par homogénéisation, de préférence d'une combinaison de mouvements de rotation verticaux et de haut en bas.

4. Procédure selon les Revendications précédentes, **caractérisée en ce que** l'extraction de l'ARN est faite par lyse et par extraction sur base de phénol, de sorte que l'ARN s'extrait même des matrices d'une concentration d'acide gras fort concentrée.

5. Procédure selon les Revendications précédentes, **caractérisée en ce que** la réaction en chaîne polymérase en temps réel est faite pour les espèces d'animaux de boeuf, mouton, et chèvre par
| | |
|---|---|
| l'amorce RTGcowM56F2a | 5'-ACC TGC GAC CTG GAG TCC T-3' |
| l'amorce RTGcowM56R2a | 5'-CTC GCG CAT CTG CCG-3' |
| TaqManmgb-sonde OptiR MGB. | 6-FAM-ACT CGT TCG TGC CGC GC- |

6. Procédure selon la Revendication 5, **caractérisée en ce que** l'amorce RTGcowM56F2a ou l'amorce RTGcowM56R2a s'applique avec la TaqManmgb-sonde OptiR.

7. Procédure selon les Revendications précédentes, **caractérisée en ce que** la réaction en chaîne polymérase en temps réel est faite pour l'espèce d'animal de cochon par les amorces suivantes
| | |
|---|---|
| Primer RTGpigM56F2 | 5'-GAC CTG CGA CGT GGA GTC CC-3' |
| Primer RTGpigM56R2 | 5'-TGG CGC TCC TCC TGC TCC-3' |
| TaqManmgb-probe OptiR | 6-FAM-ACT CGT TCG TGC CGC GC-MGB. |

8. Procédure selon la Revendication 7, **caractérisée en ce que** l'amorce RTGpigM56F2 ou l'amorce TRGpigM56R2 s'applique avec la TaqManmgb-sonde OptiR.

9. Procédure selon les Revendications précédents, **caractérisée en ce qu'**elle est effectuée dans de la viande et des produits de viande traités de chaleur.

10. Emploi de la procédure selon la Revendication 1. pour l'identification quantitative et spécifique d'espèce d'animal du tissue du système nerveux central dans la viande et des produits de viande,

11. Test kit pour l'identification quantitative et spécifique d'espèce d'animal du tissue du système nerveux central dans la viande et des produits de viande, contenant au moins les amorces
| | |
|---|---|
| RTGcowM56F2a: | 5'-ACC TGC GAC CTG GAG TCC T-3' |
| RTGcowM56R2a: | 5'-CTC GCG CAT CTG CCG-3' |
| RTGpigM56F2: | 5'-GAC CTG CGA CGT GGA GTC CC-3' |
| RTGpigM56R2: | 5'-TGG CGC TCC TCC TGC TCC-3' |
pour l'analyse spécifique d'espèce d'animal et quantitative de l'du cARN de la protéine gliofibrillaire acide PGFA.

12. Test kit pour l'identification quantitative et spécifique d'espèce d'animal du tissue du système nerveux central dans la viande et des produits de viande, selon la Revendication 11, contenant des matériaux pour l'extraction de l'ARN aussi que des tampons de réaction appropriés, et/ou des matériaux pour la transcription inverse de l'mARN extrait de la PGFA. , containing materials for RNA extraction as well as suitable reaction buffers and/or materials for the reverse transcription of the extracted GFAP mRNA.

13. Test kit pour l'identification quantitative et spécifique d'espèce d'animal du tissue du système nerveux central dans la viande et des produits selon la Revendication 12, **caractérisée en ce que** les matériaux pour l'extraction de l'RN sont de l'eau libre, des tampons transcriptase inverse (TI), MgCl2, 2'-desoxyribo-nucleoside-'5-triphosphate (dNTP), random hexamers, ARNse inhibiteurs, et transcriptase inverse.

14. Test kit pour l'identification quantitative et spécifique d'espèce d'animal du tissue du système nerveux central dans la viande et des produits selon les Revendications 11 à 13, **caractérisée en ce qu'**il contient un contrôle de transcription en forme d'un mARN de PGFA pour surveiller le procédé de transcription couronné de succès de l'mARN de PGFA en cADN.

15. Test kit pour l'identification quantitative et spécifique d'espèce d'animal du tissue du système nerveux central dans la viande et des produits selon les Revendications 11 à 14, **caractérisée en ce que** les matériaux pour la transcription inverse de l'mARN extrait de PGFA pour la détection des espèces d'animaux de boeuf, mouton, et chèvre sont Universal PCR Master, MgCl2, l'amorce RTGcowM56F2a 5'-ACC TGC GAC CTG GAG TCC T-3', l'amorce RTGcowM56R2a 5'-CTC GCG CAT CTG CCG-3', et TaqManmgb-sonde OptiR 6-FAM-ACT CGT TCG TGC CGC GC-MGB.

16. Test kit pour l'identification quantitative et spécifique d'espèce d'animal du tissue du système nerveux central dans la viande et des produits selon les Revendications 11 à 15, **caractérisée en ce que** les matériaux pour la transcription inverse de l'mARN extrait de PGFA pour la détection de l'espèce d'animal de cochon sont Universal PCR Master, MgCl2, l'amorce RTGpigM56F2 5'-GAC CTG CGA CGT GGA GTC CC-3', l'amorce RTGpigM56R2 5'-TGG CGC TCC TCC TGC TCC-3', et TaqManmgb-sonde OptiR 6-FAM-ACT CGT TCG TGC CGC GC-MGB.

17. Test kit pour l'identification quantitative et spécifique d'espèce d'animal du tissue du système nerveux central dans la viande et des produits selon les Revendications 11 - 16, **caractérisée en ce qu'**il contient un contrôle positif en forme de dADN de PGFA de l'espèce d'animal de boeuf, respectivement de cochon, et un contrôle négatif en forme de cARN de PGFA de l'espèce d'animal de boeuf, respectivement de cochon, un contrôle d'amplification interne, aussi que des essais de référence pour la quantification des essais de test examinés.

18. Test kit pour l'identification quantitative et spécifique d'espèce d'animal du tissue du système nerveux central dans la viande et des produits selon les Revendications 11 - 17, **caractérisée en ce que** les essais de référence sont des séries de dilution, des essais d'un contenu défini de tissu du système nerveux central, et/ou un gène de référence.


### SEQUENCE LISTING

<110> TransMIT Gesellschaft für Technologietransfer mbH
<120> Tierartspezifischer und quantitativer Nachweis von ZNS-Gewebe in Fleisch und Fleischerzeugnissen
<130> An.159/Bü/Sc/Ab
<140> DE 10361489.3 <141> 2003-12-23
<160> 6
<170> PatentIn version 3.1
<210> 1
   <211> 19
   <212> DNA
   <213> Artificial sequence
<220>
   <223> GFAP-gene Exon 5 and Exon 6
   <220>
   <221> Vorwärtsprimer RTGcowM56F2a
   <222> (1)..(19)
   <223> primer for determination BSE-riskmaterial from cow, sheep and goa t
<400> 1
   acctgcgacc tggagtcct 19
<210> 2
   <211> 15
   <212> DNA
   <213> Artificial Sequence
<220>
   <223> GFAP-gene Exon 5 and Exon 6
<220>
   <221> Rückwärtsprimer RTGcowM56R2a
   <222> (1)..(15)
   <223> determination of BSE-riskmaterial from cow, goat and sheep
<400> 2
   ctcgcgcatc tgccg 15
<210> 3
   <211> 17
   <212> DNA
   <213> Artificial sequence
<220>
   <223> GFAP-gene Exon 5 and Exon 6
<220>
   <221> TaqManmgb-sonde OptiR
   <222> (1)..(17)
   <223> Determination of BSE riskmaterial from cow, sheep and goat
<400> 3
   actcgttcgt gccgcgc 17
<210> 4
   <211> 20
   <212> DNA
   <213> Artificial Sequence
<220>
   <223> GFAP-gene Exon 5 and Exon 6
<220>
   <221> vorwärtsprimer RTGpigM56F2
   <222> (1)..(20)
   <223> Determination of BSE riskmaterial from pig
<400> 4
   gacctgcgac gtggagtccc 20
<210> 5
   <211> 18
   <212> DNA
   <213> Artificial Sequence
<220>
   <223> GFAP-gene Exon 5 and Exon 6
<220>
   <221> Rückwärtsprimer RTGpigM56R2
   <222> (1)..(18)
   <223> Determination of BSE riskmaterial from pig
<400> 5
   tggcgctcct cctgctcc 18
<210> 6
   <211> 17
   <212> DNA
   <213> Artificial sequence
<220>
   <223> GFAP-gene Exon 5 and Exon 6
<220>
   <221> OptiR TaqManmgb-Sonde
   <222> (1)..(17)
   <223> Determination of BSE riskmaterial from pig
<400> 6
   actcgttcgt gccgcgc 17